# EUROPEAN PATENT APPLICATION

(11) **EP 0 552 439 A1**
(43) Date of publication of application: **28.07.1993**
(21) Application number: 92119887.5
(22) Date of filing: 23.11.1992
(51) Int. Cl.: A61K 35/78

(54) **Pharmaceutical preparations for topical use in the treatment of psoriasis and atopical dermatitis**

(30) Priority: 02.12.1991 IT BG910042
(71) Applicant: VALETUDO S.r.l., Presezzo (IT); MEDIATOR s.r.l., I-20159 Milano (IT)
(72) Inventor: Bortolin, Vittorino, Presezzo (Bergamo) (IT)
(74) Representative: Lecce, Giovanni

(57) **Abstract**

Pharmaceutical preparations for topical use in the treatment of psoriasis and atopical dermatitis including silymarin or one of its active principles as the active substance and at least theophylline or related water soluble derivatives, troxerutin, beta-glycyrrhetic acid and dry extract of Ruscus Aculeatus as adjuvant substance.

The pharmaceutical preparation is in the form of creamy emulsions, ointments, lotions, gel or powder.

## Description

The present invention relates to pharmaceutical preparations for topical use. More specifically the present invention relates to pharmaceutical preparations for topical use designed for the treatment of psoriasis and atopical dermatitis.

Numerous specific preparations for the topical symptomatic treatment of the above cutaneous pathologies, i.e. psoriasis and atopical dermatitis, are known.

Said preparations call for the use of particular active substances such as retinoic acid, natural tar, some metabolites of vitamin D₃, the corticosteroids, etc.

These preparations display various action mechanisms, often unclear, and bring often unpleasant side effects which are sometimes serious such as local irritation, especially with retinoic acid, unpleasant odor, especially with mineral tar, and contraindications, especially with the corticosteroids.

The purpose of the present invention is to provide a pharmaceutical preparation for the topical symptomatic treatment of the above cutaneous pathologies which would not bring the above mentioned side effects.

In the most general aspect of the present invention said purpose is achieved by a pharmaceutical preparation employable for the topical treatment of psoriasis and atopical dermatitis containing active substances of natural origin having excellent local and systematic tolerability and free from the above mentioned side effects.

Said substances are the purified dry extract of Silybum marianum known also under the technical name of silymarin, and its active principles such as silybin, silicristin, silidianin, alone or mixed together.

The object of the present invention is therefore a pharmaceutical preparation for topical use in the treatment of psoriasis and atopical dermatitis including as the active substance the purified dry extract of Silybum marianum, known by its technical name silymarin, or one of its active principles and at least one adjuvant substance.

The silymarin and its active principles such as silybin, silicristin and silidianin can be employed alone or in a mixture together in any proportions.

The therapeutic action of the silymarin and/or its active principles in the treatment of psoriasis and atopical dermatitis takes place either when these substances are used pure or when they are used in the form of salts or phospholipidic complexs.

Among the phospholipidic complexes are recalled by way of example the silybin-cytidylphosphatidylcholine complex in the ratio in moles of 1:2, generally denominated silipide.

The quantity of active substance present in the pharmaceutical preparation of the present invention is between 0.3% and 5% by weight and preferably between 0.4% and 2% by weight of total preparation.

These substances are well known in pharmacology where they are used for purposes different from those of the present invention. Thus for example silymarin is employed due to its liver-sustaining and antioxydizing properties (radical scavenger) in preparations to be taken usually orally. The theophylline and its water soluble derivatives are used in the systemic therapy of the 'status asmathicus' resistant to the adrenergic agonists.

It is known in addition that the substances employed in the pharmaceutical preparation of the present invention and in particular the silybin possess, in addition to antioxidizing activity, a pronounced inhibiting action of the cyclic AMP phosphodiesterase enzyme. This enzyme is involved in many immunity and inflammatory processes.

It is also known that psoriasis is characterized by high cellular proliferation of the epidermis with excessive production of keratinocytes and very high cyclic AMP phosphodiesterasic activity.

Tests made by the applicant have shown that:
1. inhibition of the cyclic AMP fosfodiesterasis enzyme takes on considerable importance in the treatment of the two cutaneous pathologies psoriasis and atopical dermatitis in addition to its antioxidizing action, and that
2. considerable improvements in the care of said pathologies are achieved by applying on the areas involved topical preparations in the form of creams, ointments, lotions, gels and powders containing as the active substance silymarin or one of its active principles, e.g. silybin, in pure state or in the form of a lipophile complex.

It has also been found by the applicant that the effectiveness of the above pharmaceutical preparations is further enhanced if to the active substance is added an adjuvant substance.

The adjuvant substances shown to be particularly effective with silymarin or one of its active principles for the purpose of the present invention are theophylline; a water soluble derivative of theophylline such as diprophylline, piperazine theophylline acetate, theophylline ethylenediamine etc.; troxerutin; beta-glycyrrhetic acid, and dry extract of 'Ruscus Aculeatus'. Said adjuvant substances can be used alone or in any mixture.

The quantity of adjuvant substance to be used in the pharmaceutical preparation of the present invention can vary between 0.2% and 10% by weight, preferably between 2% and 5% of total weight of the preparation.

An example of a pharmaceutical preparation particularly effective in the treatment of psoriasis and atopical dermatitis comprises:
a) from 0.3 to 5 parts by weight of silymarin,
b) from 0.5 to 10 parts by weight of theophylline or one of its water soluble derivatives,
c) from 0.2 to 2 parts by weight of troxerutin,
d) from 0.4 to 2 parts by weight of beta-glycyrrhetic acid,
e) from 0.05 to 5 parts by weight of dry extract of Ruscus Aculeatus, and
f) inert substances with solubilizing, diluent, emulsifying and/or stabilizing action and purified water up to 100.

Preferably a pharmaceutical preparation of the present invention comprises:
a') from 0.4 to 2 parts by weight of silymarin,
b') from 2 to 5 parts by weight of theophylline or one of its water soluble derivatives,
c') from 0.5 to 1.2 parts by weight of troxerutin,
d') from 0.6 to 1.3 parts by weight of beta-glycyrrhetic acid,
e') from 0.5 to 2 parts by weight of dry extract of Ruscus Aculeatus, and
f') inert substances with solubilizing, diluent, emulsifying and/or stabilizing action and purified water up to 100.

The pharmaceutical preparation of the present invention can be in the form of creamy emulsions, ointments, lotions, gel or powder.

The inert substances with solubilizing, diluent, emulsifying and/or stabilizing action are well known and conventional and are those generally used for the preparation of pharmaceutical preparations for topical use. Some examples of such inert substances are cetylic alcohol, polyethylene glycol (having for example a molecular weight of 1000)-monocetyl ether, vaseline oil, dimethicone, propylene glycol, paraoxybenzoate of methyl, ethyl and/or propyl, dehydroacetate of sodium or potassium, etc.

To better understand the present invention and reduce it to practice, a few examples of pharmaceutical preparations are given below by way of illustration and non limiting example of the purpose of the invention.

### Example 1

Pharmaceutical preparation for topical use containing silymarin as the active substance and troxerutin and dry extract of Ruscus Aculeatus as adjuvant substances.

A cream with the following composition was prepared:

| | |
|---|---|
| Silymarin | 1.8 g |
| Troxerutin | 0.8 g |
| Dry extract of Ruscus Aculeatus | 1.1 g |
| Cetyl alcohol | 9.5 g |
| Polyethylene glycol 1000 monocetyl ether | 1.9 g |
| Vaseline oil | 10.0 g |
| Dimethicone | 0.4 g |
| Propylene glycol | 5.0 g |
| Paraoxibenzoate of methyl and propyl | 0.4 g |
| Sodium dehydroacetate | 0.15 g |
| Purified water QS | 100 g |

### Example 2

Pharmaceutical preparation for topical use containing silymarin as the active substance and troxerutin, theophylline, diprophylline and beta-glycyrrhetic acid as adjuvant substances.

A cream was prepared having the following composition:

| | |
|---|---|
| Silymarin | 1.0 g |
| Theophylline | 0.7 g |
| Diprophylline | 1.4 g |
| Troxerutin | 0.8 g |
| Acido beta-glycyrrhetic | 1.0 g |
| Cetylic acid | 9.5 g |
| Polyethylene glycol 1000 monocetyl ether | 1.9 g |
| Vaseline oil | 10.0 g |
| Dimethicone | 0.4 g |
| Propylene glycol | 5.0 g |
| Paraoxybenzoates of methyl and propyl | 0.4 g |
| Sodium dehydroacetate | 0.15 g |
| Purified water QS | 100 g |

### Example 3

Pharmaceutical preparation for topical use containing silybin as the active substance and troxerutin, diprophylline, theophylline and beta-glycyrrhetic acid as adjuvant substances.

A cream was prepared having the following composition:

| | |
|---|---|
| Silybin | 0.5 g |
| Theophylline | 1.0 g |
| Diprophylline | 1.8 g |
| Troxerutin | 0.8 g |
| Acido beta-glycyrrhetic | 1.0 g |
| Cetylic acid | 9.5 g |
| Polyethylene glycol 1000 monocetyl ether | 1.9 g |
| Vaseline oil | 10.0 g |
| Dimethicone | 0.4 g |
| Propylene glycol | 5.0 g |
| Paraoxybenzoates of methyl and propyl | 0.4 g |
| Sodium dehydroacetate | 0.15 g |
| Purified water QS | 100 g |

### Example 4

Pharmaceutical preparation for topical use including the complex silybin-cytidylphosphocoline in the molar ratio 1:1 (silipide) as the active substance and theophylline, diprophylline, troxerutin and dry extract of Ruscus Aculeatus as adjuvant substances.

A cream was prepared having the following composition:

| | |
|---|---|
| Silybin-cytidylphosphocoline complex | 1.0 g |
| Theophylline | 1.5 g |
| Diprophylline | 2.0 g |
| Troxerutin | 0.8 g |
| Dry extract of Ruscus Aculeatus | 0.9 g |
| Cetylic alcohol | 9.5 g |
| Polyethylene glycol 1000 monocetyl ether | 1.9 g |
| Vaseline oil | 10.0 g |
| Dimethicone | 0.4 g |
| Propylene glycol | 5.0 g |
| Paraoxybenzoates of methyl and propyl | 0.4 g |
| Sodium dehydroacetate | 0.15 g |
| Purified water QS | 100 g |

Ten patients affected by desquamating psoriasis on the back were treated as out patients applying once a day for thirty consecutive days a light layer of the cream having the above composition.

After one week of treatment there was noted in the majority of cases an improvement of the clinical picture, a decrease in inflammation and a clear regression of the subjective symptomology, in particular itching. On the thirtieth day the clinical picture was further improved and it was possible to suggest to the patients to continue the applications twice a week for another month.

### Example 5 (control)

Ten other patients affected with desquamating psoriasis on the back were treated with the same procedure and for the same period of time with a placebo cream, set up in the same manner as Example 4 above but without the active principle (silybin-cytidylphosphocoline).

The difference between the two treatments was significant in that the improvement of the clinical picture and subjective symptomology was negligible in the placebo group.

## Claims

1. Pharmaceutical preparation for topical use in the treatment of psoriasis and atopical dermatitis including as the active substance the purified dry extract of Silybum marianum (Carduus marianus), known by its technical name silymarin, or one of its active principles and at least one adjuvant substance.

2. Pharmaceutical preparation in accordance with claim 1 in which the active principle is selected from among silybin, silicrystine, silidianine and mixtures thereof.

3. Pharmaceutical preparation in accordance with claim 1 or 2 in which the silymarin or its active principle is in salt form.

4. Pharmaceutical preparation in accordance with claim 1 or 2 in which the silymarin or its active principle is in the form of a phospholipidic complex.

5. Pharmaceutical preparation in accordance with any one of the above claims including as the active principle the silybin-phosphatidylcholine complex in a ratio of 1:1.

6. Pharmaceutical preparation in accordance with any one of the above claims characterized in that the adjuvant substance is at least one substance selected from among theophylline, a water soluble derivative of theophylline, troxerutin, beta-glycyrrhetic acid, dry extract of Ruscus Aculeatus and mixtures thereof.

7. Pharmaceutical preparation in accordance with any one of the above claims in which the active substance is between 0.3% and 5% by weight and at least one of the adjuvant substances is between 0.2% and 10% by weight of the total weight of the preparation.

8. Pharmaceutical preparation in accordance with claim 7 in which the active substance is between 0.4% and 2% by weight and at least one of the adjuvant substances is between 2% and 5% by weight of the total weight of the preparation.

9. Pharmaceutical preparation in accordance with any one of the above claims including:
a) from 0.3 to 5 parts by weight of silymarin,
b) from 0.5 to 10 parts by weight of theophylline or one of its water soluble derivatives,
c) from 0.2 to 2 parts by weight of troxerutin,
d) from 0.4 to 2 parts by weight of beta-glycyrrhetico acid,
e) from 0.5 to 5 parts by weight of dry extract of Ruscus Aculeatus, and
f) inert substances with solubilizing, diluant, emulsifying and/or stabilizing action and purified water up to 100.

10. Pharmaceutical preparation in accordance with claim 9 including:
a') from 0.4 to 2 parts by weight of silymarin,
b') from 2 to 5 parts by weight of theophylline or one of its water soluble derivatives,
c') from 0.5 to 1.2 parts by weight of troxerutin,
d') from 0.6 to 1.3 parts by weight of beta-glycyrrhetic acid,
e') from 0.5 to 2 parts by weight of dry extract of Ruscus Aculeatus, and
f') inert substances with solubilizing, diluant, emulsifying and/or stabilizing effect and purified water up to 100.

11. Pharmaceutical preparation in accordance with any one of the above claims in the form of creamy emulsions, ointments, lotions, gel or powder.

12. The use of silymarin or one of its active principles in the treatment of psoriasis and atopical dermatitis.

13. The use of silymarin and one of its active principles together with at least one adjuvant substance selected from among theophylline, a water soluble derivative of theophylline, troxerutin, beta-glycyrrhetic acid, dry extract of Ruscus Aculeatus and mixtures thereof in the treatment of psoriasis and atopical dermatitis.
